(19) Europäisches Patentamt
European Patent Office
Office européen des brevets

(11) Publication number: **0 432 471 A1**

# (12) EUROPEAN PATENT APPLICATION

(21) Application number: **90121613.5**

(22) Date of filing: **12.11.90**

(51) Int. Cl.[5] **C07C 313/28**, C07C 313/06, C07C 311/51, A01N 41/00

(30) Priority: **16.11.89 JP 296213/89**

(43) Date of publication of application: **19.06.91 Bulletin 91/25**

(84) Designated Contracting States: **AT BE CH DE DK ES FR GB GR IT LI LU NL SE**

(71) Applicant: **MITSUI PETROCHEMICAL INDUSTRIES, LTD.**
**2-5, Kasumigaseki 3-chome Chiyoda-ku**
**Tokyo(JP)**

(72) Inventor: **Hashimoto, Isao, c/o Mitsui Petrochemical Ind.**
**Ltd, 1-2, Waki 6-chome, Waki-cho**
**Kuga-gun, Yamaguchi-ken(JP)**
Inventor: **Tsuru, Kazutaka, c/o Mitsui Petrochemical Ind.**
**Ltd, 1-2, Waki 6-chome, Waki-cho**
**Kuga-gun, Yamaguchi-ken(JP)**
Inventor: **Ishida, Tatsuyoshi, c/o Mitsui Petrochemical Ind.**
**Ltd, 1-2, Waki 6-chome, Waki-cho**
**Kuga-gun, Yamaguchi-ken(JP)**

(74) Representative: **Hansen, Bernd, Dr. Dipl.-Chem. et al**
**Hoffmann, Eitle & Partner Patent- und Rechtsanwälte Arabellastrasse 4**
**W-8000 München 81(DE)**

## (54) Novel chloroacetamide derivates and processes for the production of the same and the use thereof.

(57) A chloroacetamide derivative, useful as a herbicide, of the formula [I],

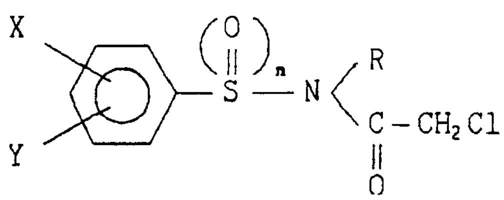

· · · [ I ]

wherein n represents 0, 1 or 2, R is hydrogen, loweralkyl, lowerhaloalkyl, cycloalkylmethyl, benzyl, substituted benzyl, loweralkoxyalkyl, tetrahydrofurfuryl, loweralkoxycarbonylmethyl, or lowerdialkylaminoethyl, and X and Y are the same or different and are hydrogen, halogen, loweralkyl, loweralkoxyl, trifluoromethyl or nitro, provided that when n = 0 and one of X and Y is p-nitro and the other is hydrogen, then R is not hydrogen, and processes for preparing the same.

EP 0 432 471 A1

# NOVEL CHLOROACETAMIDE DERIVATIVES AND PROCESSES FOR THE PRODUCTION OF THE SAME AND THE USE THEREOF

[Field of the invention]

This invention relates to a novel chloroacetamide derivative of the formula [I],

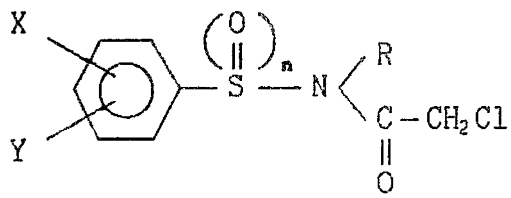

· · · [ I ]

wherein n represents 0, 1 or 2, R is hydrogen, loweralkyl, lowerhaloalkyl, cycloalkylmethyl, benzyl, substituted benzyl, loweralkoxyalkyl, tetrahydrofurfuryl, lower alkoxycarbonylmethyl, or lower dialkylaminoethyl, and X and Y are the same or different and are hydrogen, halogen, lower alkyl, lower alkoxyl, trifluoromethyl or nitro, provided that when n = 0, and one of X and Y is p-nitro and the other is hydrogen, then R is not hydrogen, and to a process for the production of the same, and also to a herbicide containing said derivative as an active ingredient.

The compounds of the formura [I] wherein n is 0, 1 and 2 correspond to those of the formulas [I′], [I″] and [I‴] respectively, of the following reaction schema (1), (2) and (3).

(1)

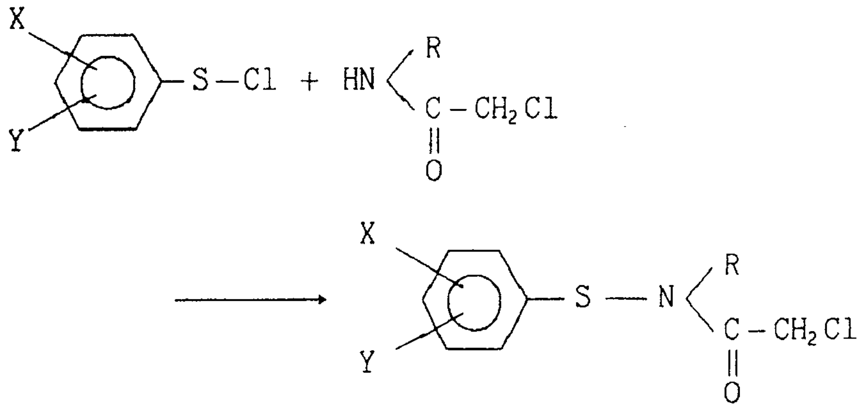

[I']

(2)

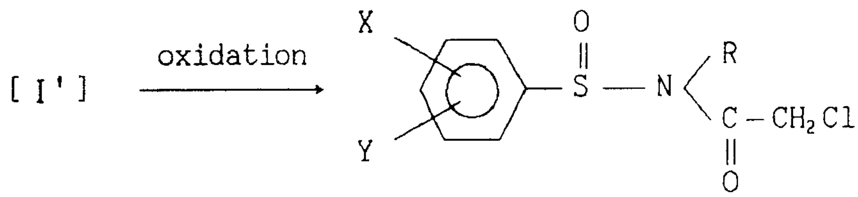


[II'']

(3)

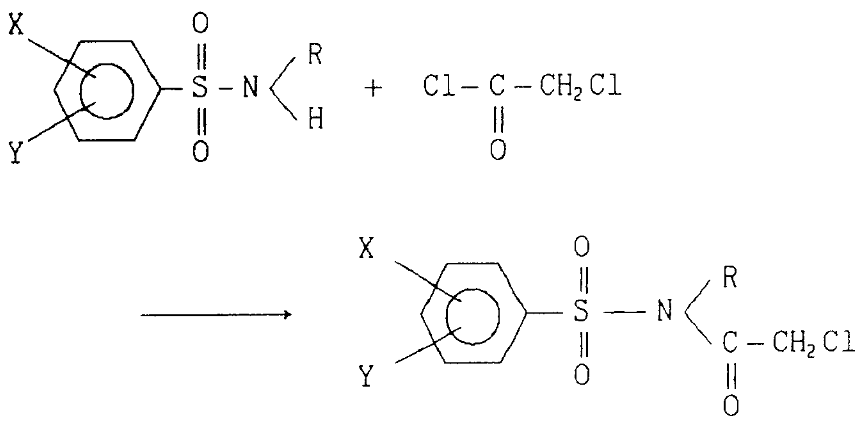

[I''']

wherein R, X and Y are as defined above.

The compounds of the invention are useful as a herbicide. They are particularly effective on the various types of annual weeds in the paddy field and upland field.

[Background of the invention]

Recently, a number of herbicides have been developed, and they have contributed to save labor and to improve the productivity in the agriculture. However, these herbicides are not necessarily satisfactory in killing weeds, and are not safe enough for use. Thus, a further improved herbicide has been requested to be developed.

[Summary of the Invention]

The inventors have found that novel chloroacetamide derivatives baving specific substituents have remarkable herbicidal effects on wededs.

The novel chloroacetamide derivatives of the invention are represented by the following formula [I].

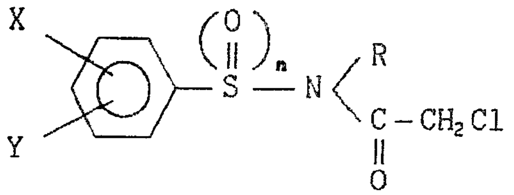

... ( I )

wherein n represents 0, 1 or 2, R is hydrogen, loweralkyl, lowerhaloalkyl, cycloalkylmethyl, benzyl, substituted benzyl, lower alkoxyalkyl, tetrahydrofurfuryl, lower alkoxycarbonylmethyl, or lower dialkylaminoethyl, and X and Y are the same or different and are hydrogen, halogen, lower alkyl, lower alkoxyl, trifluoromethyl or nitro, provided that when n = 0, and one of X and Y is p-nitro and the other is hydrogen, then R is not hydrogen.

Lower alkyls which may be mentioned include methyl, ethyl, propyl, isopropyl, butyl, sec-butyl, isobutyl, t-butyl, pentyl and the like. Haloalkyls which may be mentioned are chloromethyl, chloroethyl, chloropropyl, bromoethyl, fluoroethyl, iodoethyl, and the like. Suitable cycloalkylmethyls are cyclopropylmethyl, cyclobutylmethyl, cyclopentylmethyl, cyclohexylmethyl, and the like. Substituted benzyls are preferably chlorobenzyl, methylbenzyl, methoxybenzyl, and the like. Preferable lower alkoxyalkyls include methoxyethyl, ethoxyethyl, methoxypropyl, ethoxypropyl and the like. Preferable lower alkoxycarbonylmethyls include methoxycarbonylmethyl, ethoxycarbonylmethyl, propoxycarbonylmethyl and the like, and preferable lower dialkylaminoethyls include dimethylaminoethyl, diethylaminoethyl and the like. Halogen represents fluorine, chlorine, bromine and iodine.

The particularly preferred compounds of the invention are given in Table 1.

Table 1

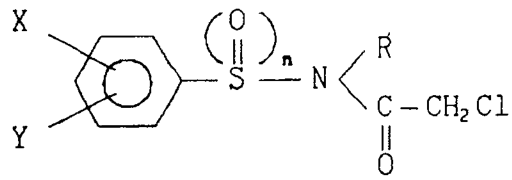

| Compound No. | X | Y | n | R |
|---|---|---|---|---|
| 1 | 3-Cl | 4-Cl | 0 | -H |
| 2 | H | 4-Cl | 0 | -H |
| 3 | H | 4-Cl | 0 | $-CH_3$ |
| 4 | H | 4-Cl | 0 | $-C_2H_5$ |
| 5 | H | 4-Cl | 0 | $-C_3H_7{}^{n}$ |
| 6 | H | 4-Cl | 0 | $-C_4H_9{}^{n}$ |
| 7 | H | 4-Cl | 0 | $-C_5H_{11}{}^{n}$ |
| 8 | H | 4-Cl | 0 | $-C_3H_7{}^{iso}$ |
| 9 | H | 4-Cl | 0 | $-C_4H_9{}^{sec}$ |
| 10 | H | 4-Cl | 0 | $-C_4H_9{}^{t}$ |
| 11 | H | 4-Cl | 0 | $-CH_2CH(CH_3)_2$ |
| 12 | H | 4-Cl | 0 | $-CH_2$ 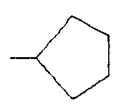 |
| 13 | H | 4-Cl | 0 | $-CH_2$ 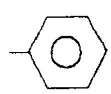 |
| 14 | H | 4-Cl | 0 | $-CH_2CH_2Cl$ |
| 15 | H | 4-Cl | 0 | $-CH_2CH_2OCH_3$ |
| 16 | H | 4-Cl | 0 | $-CH_2CH_2OC_2H_5$ |
| 17 | H | 4-Cl | 0 | $-CH_2CH_2CH_2OCH_3$ |
| 18 | H | 4-Cl | 0 | $-CH_2$ 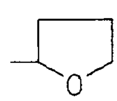 |

Table 1 ( Cont'd )

| Compound No. | X | Y | n | R |
|---|---|---|---|---|
| 19 | H | 4-Cl | 0 | $-CH_2\overset{O}{\overset{\|}{C}}OC_2H_5$ |
| 20 | H | 4-Cl | 0 | $-CH_2CH_2N(CH_3)_2$ |
| 21 | H | H | 0 | $-C_3H_7{}^n$ |
| 22 | H | 4-F | 0 | $-C_3H_7{}^n$ |
| 23 | H | $4-CH_3$ | 0 | $-C_3H_7{}^n$ |
| 24 | H | $3-CH_3$ | 0 | $-C_3H_7{}^n$ |
| 25 | H | $2-CH_3$ | 0 | $-C_3H_7{}^n$ |
| 26 | 3-Cl | 4-Cl | 0 | $-C_3H_7{}^n$ |
| 27 | 2-Cl | 5-Cl | 0 | $-C_3H_7{}^n$ |
| 28 | H | $4-NO_2$ | 0 | $-C_3H_7{}^n$ |
| 29 | H | $4-OCH_3$ | 0 | $-C_3H_7{}^n$ |
| 30 | H | $3-CF_3$ | 0 | $-C_3H_7{}^n$ |
| 31 | H | H | 1 | $-C_3H_7{}^n$ |
| 32 | H | H | 2 | $-C_3H_7{}^n$ |
| 33 | H | H | 2 | $-C_3H_7{}^n$ |

The compounds of the present invention may be prepared according to one of the following processes depending on the value of the symbolns.

(1) by reacting a compound of the formula [II]:

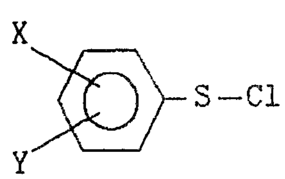

[II]

with a compound of the formula [III]

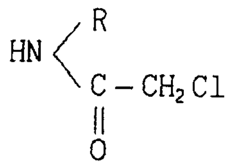

[ III ]

in the presence of a base to produce a chloroacetamide derivative of the formula [I′]

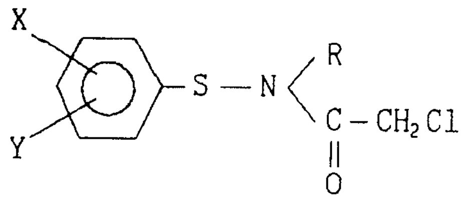

[ I′ ].

The reaction may be carried out without any solvent, or in an inert solvent such as hydrocarbons (benzene, toluene, etc.); halogenated hydrocarbons (methylene chloride, chloroform, etc.); ethers (diethyl ether, diisopropyl ether, tetrahydrofuran, etc.); and N,N-dimethylformamide (DMF), in the presence of a base such as pyridine, triethylamine, $NaHCO_3$, $Na_2CO_3$, $K_2CO_3$ and the like. The reaction temperature ranges 0° to 100° C, preferably 20° - 60° C.

(2) by oxidizing a compound of the formula [I′]

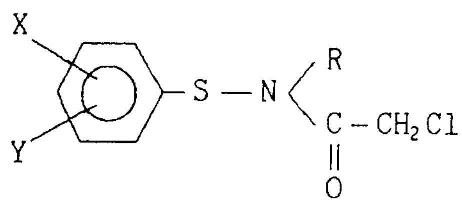

. . . [ I′ ]

to produce a chloroacetamide derivative of the formula [II″]

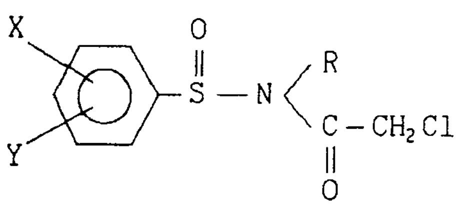

. . . [ II″ ].

This reaction is carried out using an oxidizing agent such as hydrogen peroxide, peracetic acid, perbenzoic acid, m-chloroperbenzoic acid, ozone, bromine, iodine, dinitrogen tetraoxide and the like, in a solvent such as hydrocarbons (benzene, toluene, etc.); and halogenated hydrocarbons (methylene chloride, chloroform, carbon tetrachloride, and the like). The reaction may be conducted between 0° - 50° C.

(3) by reacting a compound of the formula [IV]

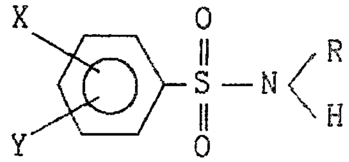

[IV]

with a compound of the formula [V]

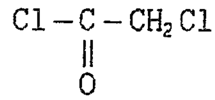

[V]

in the presence of a base to produce a chloroacetamide derivative of the formula [I'''],

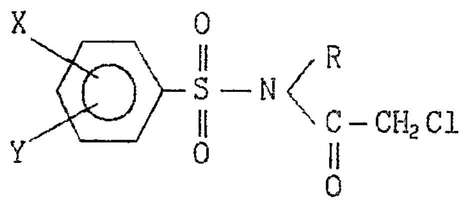

[I'''].

The reaction may be carried out without any solvent, or in a hydrocarbon solvent such as benzene, toluene, and the like; halogenated hydrocarbon such as methylene chloride, chloroform, and the like; ether such as diethylether, tetrahydrofuran, and the like, in the presence of base such as NaH, NaOH, KOH, $NaHCO_3$, $Na_2CO_3$, $K_2CO_3$, pyridine, triethylamine and the like, at a temperature of $0^\circ$ - $50^\circ C$.

The compounds of the invention may be used alone or in association with a carrier, detergents, dispersing agents, adjuvants, etc., and formulated into a suitable form such as wettable powders, emulsions, particulates or granules. The herbicide of the present invention may be applied either directly without dilution or after appropriate dilution.

The herbicide of the invention may be applied by spraying directly on weeds or by mixing with soil.

The amount of the herbicide varies depending on the type of the weeds, plants and the mode of application, and in general, about 1 -100g/are.

The effects of the compounds of the invention on weeds is illustrated referring to test data as shown below.

Test Example

The seeds of barnyard grass (Echinochloa crus-galli), crabgrass (Digitaria adscendens), rice flatsedge (Cyperus iria L), and hotarui (Scirpus juncoides Roxb) were sowed separately on double layers of filter papers in a petri dish (9cm in diameter). 20ml of a diluted liquid fertilizer, and a wettable powder to be tested (100g/10 are) were applied after dilution with water, to the petri dishes. The petri dishes were incubated with irradialion of light (3,000 lux) for 10 days at $30^\circ C$. The herbicial effects of the compounds were macroscopically evaluated accord ing to the criteria as indicated in Table 2.

Table 2

| Index Number | Herbicidal Effect ( % killed ) |
|---|---|
| 5 | 100% |
| 4.5 | 90%~99% |
| 4 | 80%~89% |
| 3.5 | 70%~79% |
| 3 | 60%~69% |
| 2.5 | 50%~59% |
| 2 | 40%~49% |
| 1.5 | 30%~39% |
| 1 | 20%~29% |
| 0.5 | 1%~19% |
| 0 | none |

The results are shown in Table 3.

Table 3

| Compound No. | Herbicidal Effect | | | |
|---|---|---|---|---|
| | barnyard glass | crabglass | rice flatsedge | hotarui |
| 2 | 2 | 1 | 2 | 4.5 |
| 3 | 5 | 5 | 3 | 2 |
| 4 | 5 | 5 | 5 | 5 |
| 5 | 5 | 5 | 5 | 5 |
| 6 | 5 | 5 | 5 | 5 |
| 8 | 5 | 5 | 5 | 5 |
| 9 | 5 | 5 | 5 | 5 |
| 11 | 5 | 5 | 5 | 5 |
| 12 | 5 | 5 | 5 | 5 |
| 14 | 5 | 5 | 5 | 3 |
| 15 | 5 | 2 | 5 | 1 |
| 18 | 5 | 3 | 5 | 3 |
| 21 | 5 | 5 | 5 | 4 |
| 22 | 5 | 5 | 5 | 4 |
| 23 | 5 | 5 | 5 | 5 |
| 24 | 5 | 5 | 5 | 5 |
| 25 | 5 | 5 | 5 | 5 |
| 26 | 5 | 5 | 5 | 5 |
| 27 | 5 | 5 | 5 | 5 |
| 28 | 5 | 5 | 5 | 2 |
| 29 | 5 | 5 | 5 | 3 |
| 30 | 5 | 4 | 5 | 3 |
| 31 | 5 | 5 | 5 | 5 |
| 32 | 3 | 2 | 4 | 3 |
| 33 | 3 | 3 | 4 | 3 |
| Control* | 0 | 0 | 0 | 0 |
| Contro2 | 0 | 0 | 0 | 0 |

* 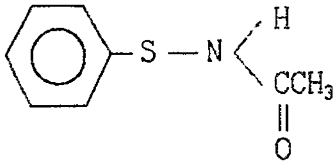 Chemical Abstracts 91 (25) 211042 g

** 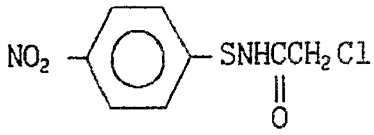 Chemical Abstracts 96 (19) 162244 s

[Effect of the invention]

The present invention provides novel chloroacetamide derivatives useful as a herbicide, and a process for producing the same. The compounds of the invention have a marked herbicidal effect on various types of annual weeds in the paddy field and upland field.

The following examples further illustrate the invention.

Example 1 Synthesis of N-(3,4-dichlorophenyl)thio-2-chloroacetamide (compound No 1)

50ml of methylene chloride, 3.1g (33 mM) of 2-chloroacetamide and 2.6g (33 mM) of pyridine were placed in 100ml flask equipped with a thermometer, a condenser, a dropping funnel and a stirrer. Then 7.1 g (33 mM) of 3,4-dichlorophenylsulfenylchloride in 10ml of methylene chloride was added dropwise over a period of 30 minutes (during this period, the temperature of the reaction mixture was elevated from 21 ° C to 33 ° C). Then, the mixture was stirred for 4 hours at 33 - 35 ° C and the solvent was distilled off under reduced pressure. The residue was poured into 200ml of water and extracted with 200ml of ethyl acetate. Ethyl acetate extract was washed with 300ml of water, dried over $MgSO_4$ and evaporated under reduced pressure. Recrystallization of the residue (7.0g of yellow solid) from toluene gave 5.4gof the title compound (yield: 60%).

m.p. : 115 ~ 116 ° C

IR(KBr disk) : $1675cm^{-1}$ ($\nu$ c = o)

MS(m/e) : 269($M^+$), 192, 177, 142

Examples 2 - 30

The compounds as shown in Table 4 were synthesized according to the procedure of Example 1 The IR data was determined in neat state when the product was liquid or in KBr disk when solid.

Table 4

| Example | Compound No. | Solvent | Yield (%) | Melting point (℃) | IR (ν c=o) (cm⁻¹) |
|---|---|---|---|---|---|
| 2 | 2 | methylene chloride | 81 | 116～118 | 1685 |
| 3 | 3 | DMF | 90 | 59～61 | 1685 |
| 4 | 4 | DMF | 80 | Liquid | 1685 |
| 5 | 5 | methylene chloride | 79 | Liquid | 1680 |
| 6 | 6 | DMF | 81 | Liquid | 1690 |
| 7 | 7 | DMF | 77 | Liquid | 1687 |
| 8 | 8 | DMF | 88 | 58～61 | 1685 |
| 9 | 9 | DMF | 90 | 56～58 | 1687 |
| 10 | 10 | DMF | 47 | Liquid | 1693 |
| 11 | 11 | DMF | 79 | Liquid | 1685 |
| 12 | 12 | Toluene | 67 | Liquid | 1685 |
| 13 | 13 | DMF | 80 | Liquid | 1690 |
| 14 | 14 | Toluene | 63 | Liquid | 1685 |
| 15 | 15 | DMF | 80 | 83～85 | 1688 |
| 16 | 16 | DMF | 84 | Liquid | 1688 |
| 17 | 17 | DMF | 87 | Liquid | 1685 |
| 18 | 18 | DMF | 77 | Liquid | 1688 |
| 19 | 19 | Toluene | 80 | Liquid | 1747, 1695 |
| 20 | 20 | DMF | 67 | 208～212 (dec.) | 1684 |
| 21 | 21 | Toluene | 83 | Liquid | 1690 |

Table 4 (cont'd)

| Example | Compound No. | Solvent | Yield (%) | Melting point (℃) | IR ($\nu$ c=o) ($cm^{-1}$) |
|---|---|---|---|---|---|
| 22 | 22 | Toluene | 68 | Liquid | 1680 |
| 23 | 23 | Toluene | 77 | Liquid | 1680 |
| 24 | 24 | Toluene | 60 | Liquid | 1690 |
| 25 | 25 | Toluene | 89 | Liquid | 1685 |
| 26 | 26 | Toluene | 79 | Liquid | 1685 |
| 27 | 27 | Toluene | 88 | Liquid | 1680 |
| 28 | 28 | Methylene choloride | 27 | Liquid | 1690 |
| 29 | 29 | Toluene | 86 | Liquid | 1675 |
| 30 | 30 | Toluene | 93 | Liquid | 1685 |

Example 31 Synthesis of N-phenylsulfenyl-N-n-propyl-2-chloroacetamide (Compound No. 31)

60ml of chloroform and 6. 6g (27 mM) of N-phenylthio-N-n-propyl-2-chloroacetamide (Compound No 21) were placed in 200ml flask equipped with a thermometer, a condenser, a dropping funnel and a stirrer. To this mixture, 8. 0g (85% purity) of m-chloroperbenzoic acid (39 mM) in 60ml of chloroform was added dropwise over a period of one-hour at 0 -5° C. The mixture was stirred for one hour at 20 - 25° C. and then filtered. The filtrate was concentrated under reduced pressure. To the residue, 200ml of satuated $NaHCO_3$ was added and the mixture was extracted with 200ml of ethyl acetate. The ethyl acetate extract was washed with satuated NaCl, dried over $MgSO_4$, and distilled under reduced pressure to give 6.7g of the title compound (Yield; 96%, pale yellow liquid).
Ir (neat) : $1685cm^{-1}$ ($\nu$c = o)
MS (m/e) : 259 ($M^+$), 219, 125

Example 32 Synthesis of N-phenylsulfonyl-N-n-propyl-2-chloroacetamide (Compound No. 32)

1.54g of sodium hydride (60% in mineral oil) (38.5 mM) was placed in 300ml flask equipped with a thermometer, a condenser, a dropping funnel, and a stirrer. The mineral oil was washed off with hexane, and 100ml of benzene was added to the flask. To the mixture, 6.97g (35.0 mM) of N-n-propylbenzenesulfonamide in 20ml of benzene was added dropwise over a period of 10 minutes at 25 - 35° C. 4.35g (38.5 mM) of chloroacetyl chloride in 10ml of benzene was added dropwise to the mixture over 10-minutes at 25 - 40° C. The resultant mixture was refluxed for 30 minutes, cooled to room temperature and 50ml of water was added. The mixture was extracted with 100ml of toluene. The toluene extract was washed with 1N NaOH, water, and then saturated NaCl, and dried over $Na_2SO_4$. The solvent was distilled off under reduced pressure, and the residue was purified by silica gel column chromatography (solvent: ethyl acetate hexane 1:9) to give 6.5g of the title compound colorless liquid (yield; 67%).
IR (neat) : $1710cm^{-1}$ ($\nu$c = o)

Example 33 Synthesis of N-(2-chlorophenyl)sulfonyl-N-n-propyl-2-chloroacetamide (Compound No. 33)

Compound 33 was obtained similarly to the procedure of Example 32 except for using N-n-propyl-2-chlorobenzenesulfonamide in place of N-n-propylbenzenesulfonamide. Yield 90%, colorless liquid.
IR (neat) : 1710cm$^{-1}$ ($\nu$c = o)

The following Examples 34 - 37 illustrate the preparation of the herbicide according to the present invention. All parts and percentages are by weight unless otherwise stated.

Example 34 Wettable powders

A wettable powder was prepared by mixing 10% the compound of the invention, 3% sodium sulfonate of a higher alcohol and 87% Kaolin uniformly.

Example 35 Emulsions

An emulsion was prepared by mixing 20% the compound of the invention, 10% polyoxyethylenealkylaryl ether, 30% cyclohexanone, and 40% xylene uniformly.

Example 36 Granules

5% the compound of the invention, 40% bentonite, 50% clay, and 5% sodium lignin sulfonate were mixed uniformly. Water was added to the mixture which was then kneaded, granulated and dried to give granules.

Example 37 Dust

3% the compound of the invention and 97% clay were mixed uniformly to give dust.

## Claims

1. A chloroacetamide derivative of the formula [I],

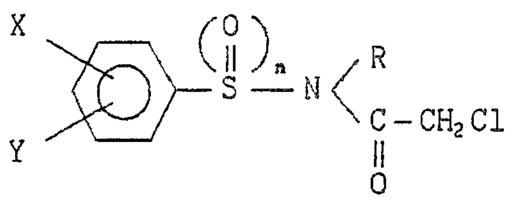

· · · [ I ]

wherein n represents 0, 1 or 2, R is hydrogen, loweralkyl, lowerhaloalkyl, cycloalkylmethyl, benzyl, substituted benzyl, lower alkoxyalkyl, tetrahydrofurfuryl, lower alkoxycarbonylmethyl, or lower dialkylaminoethyl, and X and Y are the same or different and are hydrogen, halogen, lower alkyl, lower alkoxyl, trifluoromethyl or nitro, provided that when n = 0, and one of X and Y is p-nitro and the other is hydrogen, than R is not hydrogen.

2. A method for producing a chloroacetamide derivative of the formula [I'],

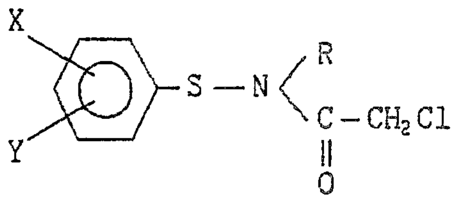

· · · [ I' ]

wherein R is hydrogen, lower alkyl, lower haloalkyl, cycloalkylmethyl, benzyl, substituted benzyl, lower alkoxyalkyl, tetrahydrofurfuryl, lower alkoxycarbonylmethyl, or lower dialkylaminoethyl, and X and Y are the same or different and are hydrogen, halogen, lower alkyl, lower alkoxyl, trifluoromethyl or nitro, provided that when one of X and Y is p-nitro and the other is hydrogen, then R is not hydrogen, comprising reacting

a compound of the formula [II],

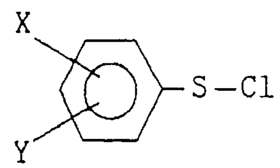

. . . [II]

wherein X and Y are as defined above, with a compound of the formula [III],

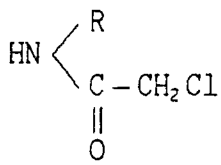

. . . [III]

wherein R is as defined above, in the presence of a base.

3. A method for producing a chloroacetamide derivative of the formula [II''],

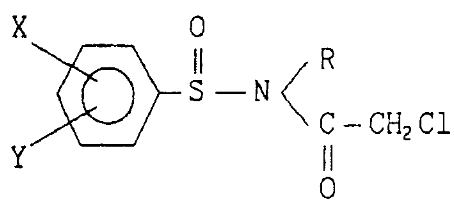

. . . [II'']

wherein R is hydrogen, lower alkyl, lower haloalkyl, cycloalkylmethyl, benzyl, substituted benzyl, lower alkoxyalkyl, tetrahydrofurfuryl, lower alkoxycarbonylmethyl, or lower dialkylaminoethyl, and X and Y are the same or different and are hydrogen, halogen, loweralkyl, lower alkoxyl, trifluoromethyl or nitro, comprising oxidizing a compound of the formula [I'],

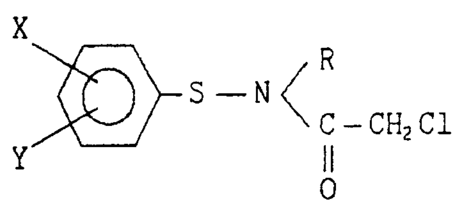

. . . [I']

where in R, X and Y are as difined above.

4. A method for producing a chloroacetamide derivative of the formula [I''']

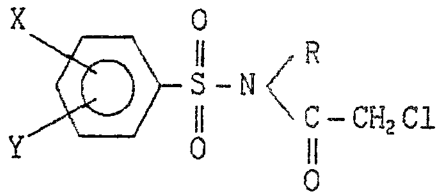

. . . [I''']

wherein R is hydrogen, lower alkyl, lower haloalkyl, cycloalkylmethyl, benzyl, substituted benzyl, lower alkoxyalkyl, tetrahydrofurfuryl, lower alkoxycarbonylmethyl, or lower dialkylaminoethyl, and X and Y are the same or different and are hydrogen, halogen, loweralkyl, loweralkoxyl, trifluoromethyl or nitro, comprising reacting a compound of the formula [IV],

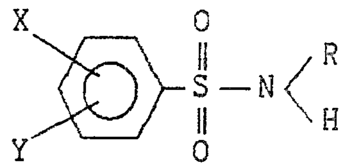

. . . [IV]

wherein R, X and Y are as difined above, with a compound of the formula [V],

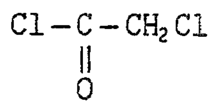

. . . [V]

in the presence of a base.

5. A herbicide comprising an effective amount of a chloroacetamide derivative as claimed in claim 1.

6. A method of killing weeds in the cultivated area which comprises applying to said area an effective amount of a chloroacetamide derivative as claimed in claim 1.

European Patent Office

# EUROPEAN SEARCH REPORT

Application Number

EP 90121613.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US - A - 4 266 078 (FERENC M. PALLOS) * Abstract; column 4, lines 36-52; table 1, compound no. 6,11,14 * -- | 1,4-6 | C 07 C 313/28<br>C 07 C 313/06<br>C 07 C 311/51<br>A 01 N 41/00 |
| D,A | CHEMICAL ABSTRACTS, vol. 96, no. 19, May 10, 1982, Columbus, Ohio, USA DUBININA, T.N. et al. "Oxidative imination of sulfene chlorides by carbocyclic N-chloramides" page 711, column 1, abstract -No. 162 244s & Zh. Org. Khim. 1982, 18(1), 162-8 -- | 1,2 | |
| D,A | CHEMICAL ABSTRACTS, vol. 91, no. 25, December 17, 1979, Columbus, Ohio, USA HARPP, DAVID N. et al. "Preparation of N-(alkylthio and -arylthio)-acetamides" page 648, column 2, abstract -No. 211 042g & J. Org. Chem., 1979, 44 (23), 4196-7 ---- | 1-3 | TECHNICAL FIELDS SEARCHED (Int. Cl.5)<br>C 07 C 313/00<br>C 07 C 311/00 |

The present search report has been drawn up for all claims

| Place of search | Date of completion of the search | Examiner |
|---|---|---|
| VIENNA | 18-02-1991 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)